(19) Europäisches Patentamt
European Patent Office
Office européen des brevets

(11) Veröffentlichungsnummer: **0 084 327**
**B1**

(12) # EUROPÄISCHE PATENTSCHRIFT

(45) Veröffentlichungstag der Patentschrift: **12.03.86**

(51) Int. Cl.⁴: **C 07 C 121/48**, C 07 C 120/00

(21) Anmeldenummer: **83100077.3**

(22) Anmeldetag: **07.01.83**

(54) **Verfahren zur Herstellung von 7,7,8,8-Tetracyanochinodimethan (TCNQ).**

(30) Priorität: **20.01.82 DE 3201485**

(43) Veröffentlichungstag der Anmeldung: **27.07.83 Patentblatt 83/30**

(45) Bekanntmachung des Hinweises auf die Patenterteilung: **12.03.86 Patentblatt 86/11**

(84) Benannte Vertragsstaaten: **CH DE FR GB IT LI**

(56) Entgegenhaltungen:
**EP-A-0 019 958**
**US-A-3 408 367**

(73) Patentinhaber: **BAYER AG, Konzernverwaltung RP Patentabteilung, D-5090 Leverkusen 1 Bayerwerk (DE)**

(72) Erfinder: **Hocker, Jürgen, Dr., Eichenweg 6, D-5060 Bergisch-Gladbach 2 (DE)**
Erfinder: **Jonas, Friedrich, Dr., Krugenofen 15, D-5100 Aachen (DE)**
Erfinder: **Merten, Rudolf, Dr., Berta-von-Suttner-Strasse 55, D-5090 Leverkusen 1 (DE)**

**EP 0 084 327 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

7,7,8,8-Tetracyanochinodimethan (TCNQ) hat in den letzten Jahren, bedingt durch seine Fähigkeit, als Elektronenakzeptor mit Elektronendonatoren Charge-Transfer-Komplexe von hoher elektrischer Leitfähigkeit zu bilden, in der Technik wachsende Bedeutung erlangt [s. D. Acker, W. Hertler, J. Am. Chem. Soc. *84*, 3374, 1962]. Das beste bisher bekannte Verfahren zur Herstellung des TCNQ besteht in der Oxidation von 1,4-Bis-(dicyanomethylen)-cyclohexan (Formel I).

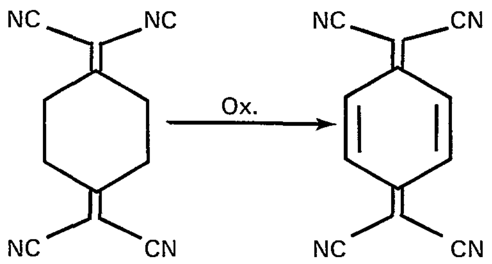


(I)

TCNQ

Als Oxidationsmittel sind Brom/Pyridin, N-Bromsuccinimid und N-Chlorsuccinimid [s. D. Acker, W. Hertler, J. Am. Chem. Soc. *84*, 3370 (1962)] beschrieben worden. Das so erhaltene 7,7,8,8-Tetracyanochinodimethan ist aber durch grössere Mengen Halogen verunreinigt.

Es wurde gefunden, dass 1,4-Bis-(dicyano-methylen)-cyclohexan mit Chlor in Gegenwart von Basen zu 7,7,8,8-Tetracyanochinodimethan von hoher Reinheit oxidiert werden kann.

Die US-Patentschrift 3 408 367 beschreibt die Herstellung von Tetra-substituierten 1,4-Bis-(dicyanomethylen)-cyclohexadien, also Tetracyanochinodimethanen, in denen alle freien Positionen des 6-Ringes mit Phenylresten substituiert sind, die ihrerseits noch weiter substituiert sein können. Diese Produkte können durch Oxidation entsprechender Vorstufen mit Chlor erhalten werden. Es war weder vorauszusehen, dass diese Reaktion auch mit den unsubstituierten Körpern in besonders vorteilhafter Weise gelingt, noch bestand bei den substituierten Produkten das Problem, halogenfreie Körper herzustellen.

Die Oxidation wird vorzugsweise in einem organischen oder anorganischen Lösungsmittel durchgeführt. Geeignete Lösungsmittel sind: Kohlenwasserstoffe wie z.B. Cyclohexan, n-Hexan, Heptan, Chlorkohlenwasserstoffe, z.B. Chloroform, Methylenchlorid, Tetrachlorkohlenstoff, 1,1,2-Trichlorethan, Alkancarbonsäurenitrile wie z.B. Acetonitril, Propionsäurenitril. Besonders bevorzugt wird Acetonitril verwendet.

Bezogen auf eingesetztes 1,4-Bis-(dicyanomethylen)-cyclohexan, können 2 bis 40, vorzugsweise 5-10 Gew.-Teile Lösungsmittel verwendet werden.

Für das Verfahren geeignete Basen sind z.B. organische Basen wie aliphatische Amine, z.B. Diethylamin, Triethylamin; aromatische Amine, z.B. Anilin, N,N-Dimethylanilin; stickstoffhaltige Heterocyclen, z.B. Pyridin, Chinolin und anorganische Basen wie Alkali- und Erdalkalicarbonate, z.B. Natriumcarbonat, Kaliumcarbonat, Calciumcarbonat, Bariumcarbonat oder Ammoniak. Besonders bevorzugt ist Pyridin.

Bezogen auf 1,4-Bis-(dicyanomethylen)-cyclohexan, können 3,5-6 mol, vorzugsweise 3,8-5 mol, insbesondere 3,9-4,2 mol organische oder anorganische Base eingesetzt werden.

In das Reaktionsgemisch aus Lösungsmittel, Base und 1,4-Bis-(dicyanomethylen)-cyclohexan können 4,0-6,0 mol, vorzugsweise 4,0-4,2 mol gasförmiges Chlor, bezogen auf eingesetztes 1,4-Bis-(dicyanomethylen)cyclohexan , eingeleitet oder eine äquivalente Menge einer Chlorlösung in einem geeigneten Lösungsmittel zugetropft werden.

Die Oxidations wird im allgemeinen im Temperaturbereich von 0-40°C, vorzugsweise 15-30°C, insbesondere 20-25°C durchgeführt. Die Reaktionszeiten liegen zwischen wenigen Minuten und mehreren Stunden.

Die Ausbeuten liegen zwischen 85 und über 90% der Theorie, bezogen auf eingesetztes 1,4-Bis-(dicyanomethylen)-cyclohexan. Das Reaktionsprodukt enthält nur geringe Mengen an halogenhaltigen Verunreinigungen.

*Beispiel 1*

In eine Suspension aus 3120 Vol.-Teilen Acetonitril, 416 Gew.-Teilen 1,4-Bis-(cyanomethylen)-cyclohexan und 672 Vol.-Teilen Pyridin werden innerhalb 7 Stunden 299 Gew.-Teile Chlor bei 20-25°C unter Rühren eingeleitet. Anschliessend wird 30 Minuten nachgerührt.

Der Feststoff wird abgesaugt, mit Acetonitril gewaschen und getrocknet. Es werden 450,9 Gew.-Teile (86% d. Theorie) Produkt erhalten, das praktisch chloriert ist und durch Sublimation weiter gereinigt werden kann.

$C_{12}H_4N_4$ (204)

| | | | |
|---|---|---|---|
| Ber.: | 70,6% C | 2,0% H | 27,4% N |
| Gef.: | 70,7% C | 1,8% H | 27,3% N |

*Beispiel 2*

Wie im Beispiel 1 beschrieben, werden 10,4 Gew.-Teile 1,4-Bis-(cyanomethylen)-cyclohexan, 60 Vol.-Teile Acetonitril, 16,8 Vol.Teile Pyridin und 7,5 Gew.-Teile Chlor bei 20-25°C zur Reaktion gebracht und aufgearbeitet. Es werden 9,2 Gew.-Teile (90% d.Th.) reines TCNQ erhalten.

**Patentansprüche**

1. Verfahren zur Herstellung von 7,7,8,8-Tetracyanochinodimethan, dadurch gekennzeichnet, dass 1,4-Bis-(dicyanomethylen)-cyclohexan mit Chlor in Gegenwart von Basen oxidiert wird.

2. Verfahren nach Anspruch 1, dadurch gekennzeichnet, dass die Oxidation in einem Lösungsmittel durchgeführt wird.

3. Verfahren nach Ansprüchen 1 und 2, dadurch gekennzeichnet, dass die Reaktion im Temperaturbereich von 0 bis 40°C durchgeführt wird.

4. Verfahren nach Ansprüchen 1 bis 3, dadurch

gekennzeichnet, dass als organische Base Pyridin verwendet wird.

5. Verfahren nach Ansprüchen 1 bis 4, dadurch gekennzeichnet, dass als Lösungsmittel Acetonitril verwendet wird.

**Claims**

1. A process for the production of 7,7,8,8-tetracyanoquinodimethane, characterised in that 1,4-bis-(cyanomethylene)-cyclohexane is oxidized with chlorine in the presence of a base.

2. A process according to claim 1, characterised in that the oxidation is carried out in a solvent.

3. A process according to claim 1 and 2, characterised in that the reaction is carried out in the temperature range of from 0 to 40°C.

4. A process according to claims 1 to 3, characterised in that pyridine is used as organic base.

5. A process according to claims 1 to 4, characterised in that acetonitrile is used as solvent.

**Revendications**

1. Procédé de production de 7,7,8,8-tétracyanoquinodiméthane, caractérisé en ce qu'on oxyde du 1,4-bis-(dicyanométhylène)-cyclohexane avec du chlore en présence de bases.

2. Procédé suivant la revendication 1, caractérisé en ce qu'on conduit l'oxydation dans un solvant.

3. Procédé suivant les revendications 1 et 2, caractérisé en ce qu'on conduit la réaction dans l'intervalle de température de 0 à 40°C.

4. Procédé suivant les revendications 1 à 3, caractérisé en ce qu'on utilise la pyridine comme base organique.

5. Procédé suivant les revendications 1 à 4, caractérisé en ce qu'on utilise l'acétonitrile comme solvant.